# EUROPEAN PATENT APPLICATION

(11) **EP 4 517 765 A1**
(43) Date of publication of application: **05.03.2025**
(21) Application number: 23796338.4
(22) Date of filing: 24.04.2023
(51) Int. Cl.: G16H 20/10

(54) **INFORMATION PROCESSING SYSTEM, INFORMATION PROCESSING METHOD, AND PROGRAM**

(30) Priority: 28.04.2022 JP 2022074508
(71) Applicant: Mitsubishi Tanabe Pharma Corp, Osaka-shi, Osaka 541-8505 (JP)
(72) Inventor: SAKATA Takeshi, Osaka-shi, Osaka 541-8505 (JP); OGUMA Takahiro, Osaka-shi, Osaka 541-8505 (JP)
(74) Representative: Hasegawa, Kan
(86) International application number: PCT/JP2023/016158
(87) International publication number: WO 2023/210598

(57) **Abstract**

[Problem] An information processing system, which can present medication notification according to medicine for which the dietary restriction period varies depending on a type of meal (meal content) by presenting medication notification based on a dietary restriction period, is provided. Furthermore, the system can also have with various management functions such as nutritional management.

[Solution] An information processing system of the present embodiment comprises a dietary restriction period setting unit configured to set a dietary restriction period according to meal-related information regarding a predetermined meal of a first user, and a medication notification presentation unit configured to present a medication notification based on mealtime information indicating mealtime when the predetermined meal is ingested and the set dietary restriction period.

## Description

### [TECHNICAL FIELD]

The present disclosure relates to an information processing system, an information processing method, and a program.

### [BACKGROUND]

Neuromuscular disease is a general term for diseases that cause movement disorders due to lesions in the nerves themselves, such as the brain, spinal cord, or peripheral nerves, or lesions in the muscles themselves. Representative diseases of the neuromuscular disease include Parkinson's disease, spinocerebellar degeneration, amyotrophic lateral sclerosis, neuritis and myelitis caused by viruses or bacteria, myasthenia gravis, muscular dystrophy, polymyositis, or the like.

Among neuromuscular diseases, for example, amyotrophic lateral sclerosis (hereinafter also referred to as "ALS") is a rapidly progressing, fatal, severe disease in which voluntary movement is impaired due to selective degeneration and loss of upper and lower motor neurons, and weakness of the upper and lower limbs, bulbar paralysis, and respiratory muscle paralysis progress gradually, often requiring respiratory management due to respiratory failure 2 to 5 years after onset.

Although oral medications are sometimes prescribed for ALS patients, the compliance rate for taking oral medications is generally low. For example, Patent Literature 1 discloses a device for managing medication that notifies the medication time when it is time to take medication.

### [PRIOR ART LIST]

### [PATENT LITERATURE]

Patent Literature 1: Japanese Unexamined Patent Application Publication No.2014-194716

### [SUMMARY]

### [TECHNICAL PROBLEM]

However, it cannot be said that the conventional medication management system sufficiently considers medication management for ALS patients, and a medication management system that responds to medication environment of the ALS patients is desirable.

In addition, there is a growing need for a system that integrates not only medication management but also various patient-related management such as symptom management and nutritional management.

### [TECHNICAL SOLUTION]

An object of the present disclosure is to provide an information processing system capable of responding to the medication environment of the ALS patient.

The main invention of the present disclosure for solving the above problem, comprises: a dietary restriction period setting unit configured to set a dietary restriction period according to meal-related information regarding a predetermined meal of a first user, and a medication notification presentation unit configured to present a medication notification based on mealtime information indicating mealtime when the predetermined meal is ingested and the dietary restriction period.

Other problems and solutions disclosed in the present application will be clarified by the description of embodiments of the invention and the drawings.

### [ADVANTAGEOUS EFFECTS]

According to the present invention, it is possible to provide the information processing system capable of responding to the medication environment of the ALS patient, in particular.

### [BRIEF DESCRIPTION OF THE DRAWINGS]

FIG. 1 is a diagram showing an example of a configuration of the information processing system including an information processing apparatus.
FIG. 2 is a diagram showing an example of a hardware configuration of the information processing apparatus.
FIG. 3 is a diagram showing an example of a software configuration of the information processing apparatus.
FIG. 4 is a diagram showing an example of a configuration of user basic information stored in a user information storage unit.
FIG. 5 is a diagram showing a flow of processing executed in the information processing apparatus.

### [DETAILED DESCRIPTION OF THE PREFERRED EMBODIMENT]

The present invention will be described below by listing contents of embodiments of the present disclosure. For example, the present disclosure has the following configuration.

### [Item 1]

An information processing system comprises:
a dietary restriction period setting unit configured to set a dietary restriction period according to meal-related information regarding a predetermined meal of a first user, and
a medication notification presentation unit configured to present a medication notification based on mealtime information indicating mealtime when the predetermined meal is ingested and the set dietary restriction period.

### [Item 2]

The information processing system according to item 1, wherein the meal-related information includes at least one of a plurality of meal type information indicating degree of the predetermined meal.

### [Item 3]

The information processing system according to item 2, wherein
the meal-related information includes meal content information indicating a content of the predetermined meal,
the meal content information is information of meal menu or ingredient set by an input operation or a selection operation by the first user or a related user related to the first user, or information of meal menu or ingredient estimated from an acquired image, and
the meal type information is set based on the information of meal menu or ingredient.

### [Item 4]

The information processing system according to item 3, wherein the related user is at least one among a family member of the first user, a caregiver who cares for the first user, a medical professional involved with the first user, and a representative who acts on behalf of the first user.

### [Item 5]

The information processing system according to any one of items 2 to 4, wherein the dietary restriction period setting unit sets the dietary restriction period according to the degree of the predetermined meal indicated by the meal type information.

### [Item 6]

The information processing system according to any one of items 1 to 5, wherein the medication notification presentation unit presents the medication notification at a time of at least one among a first time when the dietary restriction period has elapsed from the mealtime indicated by the mealtime information, a second time before the first time by a predetermined period, and a third time after the first time by a predetermined period.

### [Item 7]

The information processing system according to any one of items 1 to 6, further comprising a nutritional management target numerical value output unit configured to acquire physical information of the first user and ALSFRS-R evaluation information of the first user and output a nutritional management target numerical value including a recommended nutritional intake suited to the first user.

### [Item 8]

The information processing system according to item 7, further comprising:
a nutritional intake information generation unit configured to generate nutritional intake information from a content of the predetermined meal, and
a comparison result generation unit configured to compare at least the nutritional intake information and the recommended nutritional intake to generate a comparison result.

### [Item 9]

The information processing system according to any one of items 1 to 8, wherein the medication notification presentation unit does not present the medication notification during a predetermined non-notification period in case that medication taken information is set.

### [Item 10]

An information processing method executed on a computer, the method comprising:
a step of setting a dietary restriction period according to meal-related information related to a predetermined meal of a first user by a dietary restriction period setting unit, and
a step of presenting a medication notification based on mealtime information indicating mealtime when the predetermined meal is ingested and the dietary restriction period by a medication notification presentation unit.

### [Item 11]

A program for performing on a computer,
a dietary restriction period setting function for setting a dietary restriction period according to meal-related information related to a predetermined meal of a first user, and
a medication notification presentation function for presenting a medication notification based on mealtime information indicating mealtime when the predetermined meal is ingested and the dietary restriction period.

### <Details of the embodiment>

A specific example of an information processing apparatus 10 according to one embodiment of the present disclosure will be described with reference to the drawings. The embodiment described below does not unreasonably limit the contents of the present disclosure described in the claims. Also, not all of the components shown in the embodiments are essential components of the present disclosure. In addition, the features shown in each embodiment can be applied to other embodiments as long as they do not contradict each other.

### <Configuration>

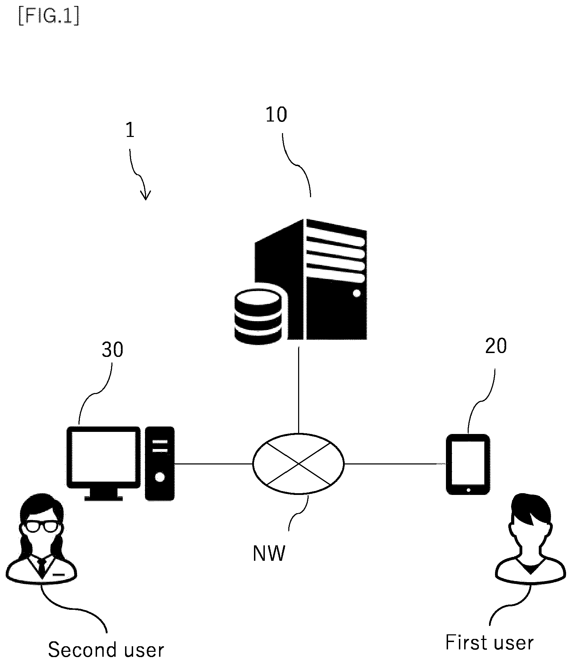

FIG. 1 is a diagram illustrating an example of an information processing system 1 including the information processing apparatus 10 according to the present embodiment. In the present embodiment, the information processing apparatus 10 is communicatively connected to a first user terminal 20 used by a first user such as a patient via a network NW. If necessary, a second user terminal 30 used by a second user such as a doctor may also be communicatively connected to the information processing apparatus 10 via the network NW.

In the present embodiment, the network NW is, for example, the internet, which is constructed by a public telephone line network, a mobile phone line network, a wireless communication network, Ethernet (registered trademark), or the like.

The information processing apparatus 10 may be a so-called server terminal, and constitutes a part of the information processing system 1 by executing information processing with the first user terminal 20 and/or the second user terminal 30 via the network NW. The information processing apparatus 10 may be, for example, a workstation or a general-purpose computer such as a personal computer, or may be logically realized by cloud computing. In the information processing apparatus 10, an application or the like capable of communicating with the first user terminal 20 and/or the second user terminal 30, or a browser for accessing a web service that enables the communication may be installed.

The first user terminal 20 is a terminal which the first user (i.e., a target person (especially the patient, etc.) who is a target of various management functions including a medication management function by the present system, etc.) mainly uses for inputting data or the like, and executes information processing with the information processing apparatus 10 and the second user terminal 30 via the network NW. The first user terminal 20 may be, for example, a general-purpose computer such as a workstation or a personal computer, or may be a mobile communication device such as a smartphone or a tablet device. The first user terminal 20 may also be a digital device such as a wearable device worn by the user. On the first user terminal 20, an application or the like that enables communication with the information processing apparatus 10 and/or the second user terminal 30, or a browser for accessing a web service that enables the communication may be installed. The first user terminal 20 may also be a smartphone that the user originally owns, or a terminal given to the user by a hospital, as long as the user inputs data. Moreover, a person inputting information to the first user terminal 20 is not limited to the user himself/herself, but may be a related user such as a family member of the user, a caregiver who cares for the user , a medical professional involved with the user, or a representative who acts on behalf of the user, or the first user terminal 20 may be a terminal used by the related user and may include a terminal into which information about the user as a subject (such as the patient, etc.) is input. The related user may be identified by related user identification information, and a related user account may be assigned to each related user. The related user identification information or the related user account may be associated with user identification information or user account of the user as the subject.

The second user terminal 30 is a terminal which the second user such as a doctor working at a medical institution such as a hospital, an insurance company, a pharmaceutical company, a patient group, a research institute, or a financial institution, uses for grasping situation of the first user, and executes information processing with the information processing apparatus 10 and/or the first user terminal 20 via the network NW. The second user terminal 30 may be, for example, a general-purpose computer such as a workstation or a personal computer, or may be a mobile communication device such as a smartphone or a tablet device. On the second user terminal 30, an application or the like that enables communication with the information processing apparatus 10 and/or the first user terminal 20, or a browser for accessing a web service that enables the communication may be installed.

A control unit of the first user terminal 20 or the second user terminal 30 can execute all or part of functions executed by a control unit 11 of the information processing apparatus 10, and all or part of information stored in a storage unit provided in the information processing apparatus 10 can be stored in a storage unit built in the first user terminal 20 or the second user terminal 30. Conversely, the control unit 11 of the information processing apparatus 10 can execute all or part of the functions by the control unit of the first user terminal 20 or the second user terminal 30, and all or part of the information stored in the storage unit provided in the first user terminal 20 or the second user terminal 30 can be stored in a storage unit built in the information processing apparatus 10.

### <Hardware Configuration>

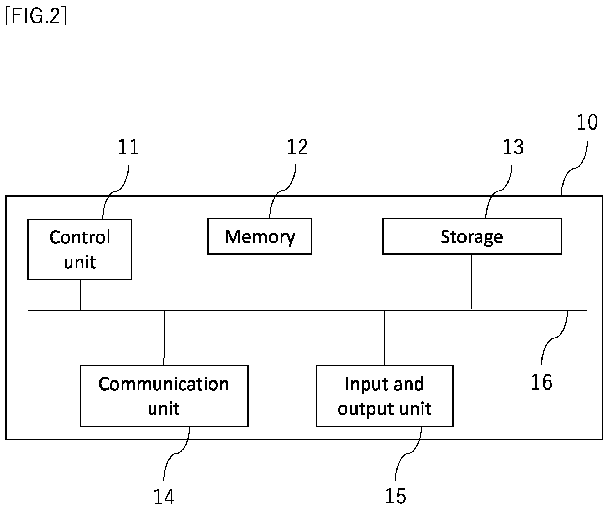

FIG.2 is a diagram showing an example of the hardware configuration of a computer that realizes the information processing apparatus 10 according to the present embodiment. The computer includes at least the control unit 11, a memory 12, a storage 13, a communication unit 14, and an input and output unit 15. These are electrically connected to each other via a bus 16.

The control unit 11 is a computing device that controls operation of the entire information processing apparatus 10 and performs information processing, etc. necessary for control of transmission and reception of data between each element, and execution and authentication processing of an application. For example, the control unit 11 is a processor such as a CPU (Central Processing Unit), and executes programs, etc. stored in the storage 13 and deployed in the memory 12 to perform each information processing.

The memory 12 includes a main memory configured with a volatile storage device such as a Dynamic Random Access Memory (DRAM) and an auxiliary memory configured with a non-volatile storage device such as a flash memory or a Hard Disc Drive (HDD). The memory 12 is used as a work area, etc. of the control unit 11 and stores a basic input/output system (BIOS) executed when the information processing apparatus 10 is started up, various setting information, or the like.

The storage 13 stores various programs such as application programs, etc. A database that stores data used in each process may be constructed in the storage 13.

The communication unit 14 connects the information processing apparatus 10 to a network. The communication unit 14 communicates with an external device directly or via a network access point, for example, by a type of a wired LAN (Local Area Network), a wireless LAN, Wi-Fi (Wireless Fidelity, registered trademark), infrared communication, Bluetooth (registered trademark), short-distance or non-contact communication, or the like.

The input and output unit 15 is, for example, an information input device such as a keyboard, a mouse, or a touch panel, etc., and an output device such as a display.

The bus 16 is commonly connected to each of the above elements and transmits, for example, address signals, data signals and various control signals.

In the present embodiment, the hardware configuration of a terminal such as a computer or a smartphone that realizes the first user terminal 20 and the second user terminal 30 is similar to the hardware configuration example of the information processing apparatus 10 shown in FIG.2, so the description will be omitted.

### <Software configuration>

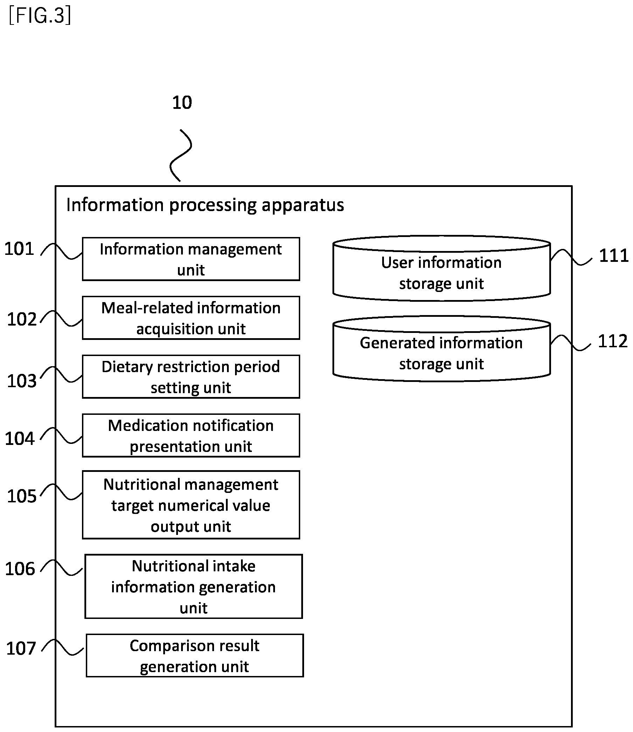

FIG.3 is a diagram showing an example of a software configuration of the information processing apparatus 10 according to the present embodiment. The information processing apparatus 10 can include functional units, of an information management unit 101, a meal-related information acquisition unit 102, a dietary restriction period setting unit 103, a medication notification presentation unit 104, a nutritional management target numerical value output unit 105, a nutritional intake information generation unit 106, and a comparison result generation unit 107, as well as storage units, of a user information storage unit **111** and a generated information storage unit 112.

The information management unit 101, the meal-related information acquisition unit 102, the dietary restriction period setting unit 103, the medication notification presentation unit 104, the nutritional management target numerical value output unit 105, the nutrient intake information generation unit 106 , and the comparison result generation unit 107 are realized by the control unit **11** provided in the information processing apparatus 10 which reads out programs stored in the storage 13 into the memory 12 and executes them. The user information storage unit **111** and the generated information storage unit 112 are realized as part of a storage area provided by the memory 12, the storage 13, or the memory 12 and the storage 13.

The information management unit 101 reads out predetermined information from various storage units and transmits it to the outside (e.g., the first user terminal 20 or the second user terminal 30), receives predetermined information from the outside and stores it in a corresponding storage unit, and stores information generated by various functional units in corresponding storage units, etc.

The meal-related information acquisition unit 102 acquires meal-related information indicating information on a predetermined meal of the first user. The meal-related information may be acquired, for example, by receiving meal-related information inputted at the first user terminal 20. The meal-related information may include mealtime information indicating mealtime when the predetermined meal is ingested, and the mealtime information may be associated with information about other meals. The acquired meal-related information, for example, may be stored in the generated information storage unit 112 in association with first user identification information of the sender.

The meal-related information may include, for example, a plurality of meal type information indicating degree of the predetermined meal. The plurality of meal type information may be meal type information classified in stages according to degree of nutritional component amount such as calorie amount and fat content, such as "high-fat meal (1000 kcal, 50% fat)", "low-fat (normal) meal (400 kcal, 25% fat)", "light meal (e.g., nutritional supplement (250 kcal))". The meal type information may be set to any one of the plurality of meal type information by an input operation or selection operation by a user on the application of the first user terminal 20, or may be set to any one of the plurality of meal type information by estimation from a meal image acquired by the first user terminal 20 by image analysis or the like. The image analysis may be performed by an analysis unit (not shown in Figures) provided in the information processing apparatus 10 (or the first user terminal 20) in a known method. The set meal type information may be acquired by the meal-related information acquisition unit 102.

The meal-related information may include, for example, meal content information indicating contents of the predetermined meal. The meal content information may be set by the input operation or the selection operation by the user on the application of the first user terminal 20, or may be set by estimating by image analysis or the like from the meal image acquired by the first user terminal 20. The image analysis may be performed by an analysis unit (not shown in Figures) provided in the information processing apparatus 10 (or the first user terminal 20) using a known method. The meal content information may include menu information and ingredient information for each meal. The menu information may be information indicating a specific menu such as hamburger steak, curry, miso soup, etc., or meal category information classified in stages according to the degree of nutritional component amount such as calorie amount and fat content, such as "high-fat meal (1000 kcal, 50% fat) menu", "low-fat (normal) meal (400 kcal, 25% fat) menu", and "light meal (e.g., nutritional supplement (250 kcal))". The ingredient information may be information indicating ingredients used in a meal, for example. Furthermore, based on the set meal content information, the above-mentioned meal type information may be set according to the degree of nutritional component amount calculated, for example, by adding amounts of nutritional components such as the caloric amount and fat content corresponding to the meal content.

The following describes in detail the "high-fat meal (1000 kcal, 25% fat)", "low-fat (normal) meal (400 kcal, 25% fat)" and "light meal (e.g., nutritional supplement (250 kcal))" exemplified in the present disclosure. The predetermined meal can be classified into one of the above categories based on, for example, general classification standards in the medical field. Specifically, for example, these categories can be classified according to total calories of one meal, and more specifically, for example, these categories can be classified according to at least one among the total calories and the calories ingested from lipids out of the total calories.

The high-fat meal (1000 kcal, 50% fat) may refer to a meal with total calories of 1000 kcal per meal and 50% of calorie intake from lipids, or a meal with total calories of 1000 kcal and 500 kcal intake from lipids. The high-fat meal (1000 kcal, 50% fat) may also refer to a meal with total calories of more than 500 kcal. Preferably, the high-fat meal may refer to a meal with total calories of 800 kcal or more and 1000 kcal or less, and 50% of calorie intake from lipids. Alternatively, the high-fat meal may refer to a meal with total calories of 800 kcal or more and 1000 kcal or less, and 500 kcal or more 600 kcal or less intake from lipids. Alternatively, the high-fat meal may refer to a meal with total calories of 800 kcal or more 1000 kcal or less, and 500 kcal or more 600 kcal or less intake from lipids, and 250 kcal intake from carbohydrate. Furthermore, when either of the following conditions is satisfied: (Condition A) total calories are greater than 500 kcal and 5000 kcal or less, or (Condition B) calorie intake from lipids is greater than 125 kcal and 5000 kcal or less, it can also be classified as a high-fat meal (1000 kcal, 50% fat). A range of total calories of (Condition A) can be, for example, greater than 500 kcal and 5000 kcal or less, preferably 800 kcal or more and 2000 kcal or less, more preferably 800 kcal or more and 1500 kcal or less, and even more preferably 800 kcal or more and 1000 kcal or less. In addition, the calorie intake from lipids of (Condition B) can be, for example, greater than 125 kcal and 5000 kcal or less, preferably 200 kcal or more and 1000 kcal or less, more preferably 400 kcal or more and 750 kcal or less, and even more preferably 500 kcal or more and 600 kcal or less. In the present disclosure, the high-fat meal (1000 kcal, 50% fat) may be a meal described as a high-fat meal in the FDA guidance (Assessing the Effects of Food on Drugs in INDs and NDAs -Clinical Pharmacology Considerations), or may be a meal described as a high-fat meal in the EMA Guideline (Guideline in the investigation of drug interactions).

The low-fat (normal) meal (400 kcal, 25% fat) may refer to a meal with total calories of 400 kcal per meal and 25% of calorie intake from lipids, or a meal with total calories of 400 kcal and 100 kcal intake from lipids. In addition, a meal that satisfies the following conditions can also be classified as a low-fat (normal) meal (400 kcal, 25% fat): (Condition C) total calories are greater than 250 kcal and 500 kcal or less, and calorie intake from lipids is less than 100 kcal, (Condition D) total calories are 250 kcal or less, and calorie intake from lipids is 100 kcal or more and 125 kcal or less, or (Condition E) total calories are greater than 250 kcal and 500 kcal or less, and calorie intake from lipids is 100 kcal or more and 125 kcal or less. In the present disclosure, the low-fat (normal) meal (400 kcal, 25% fat) may be a meal described as a low-fat meal in the FDA guidance (Assessing the Effects of Food on Drugs in INDs and NDAs -Clinical Pharmacology Considerations). In the present disclosure, the low-fat (normal) meal (400 kcal, 25% fat) may be referred to as a moderate meal.

An example of the light meal is a liquid type nutritional supplement (enteral nutrient: Ensure Liquid (registered trademark)) of about 250 kcal. In addition, a meal that satisfies (Condition F) total calories are greater than 0 kcal and 250 kcal or less, and (Condition G) the calories ingested from lipids are greater than 0 kcal and less than 100 kcal, can also be classified as the light meal. In this disclosure, the light meal may be referred to as calorie aid food, calorie supplementary food, calorie supplementary agent, nutritional aid food, nutritional supplementary agent, nutritional food, enteral nutrient, protein amino acid preparation, elemental diet, oligomeric enteral nutrient, oligomeric formula, polymeric enteral nutrient, polymeric formula, nutritional supplementary food, nutritional supplementary drink, nutritional food, or nutritional functional food.

In the present embodiment, "kilocalorie", which is a unit of total calories, is defined as 1 kilocalorie of energy required to raise the temperature of 1 liter of water by 1°C. Furthermore, the kilocalorie in the present specification is synonymous with the "calories" used in meals as described on the website of the United States Department of Agriculture (USDA) (https://www.nutrition.gov/expert-qa (see the section "What is the difference between calories and kilocalories")). As described above, in the present specification, the unit "kilocalorie" is based on the former definition. For example, 800 kcal or more and 1000 kcal or less described in the present specification means 800 or more and 1000 or less calories according to the explanation of the United States Department of Agriculture (USDA).

The dietary restriction period setting unit 103 sets dietary restriction period information indicating a dietary restriction period according to meal-related information indicating information on dietary content. The dietary restriction period is a period set after mealtime when a medicine scheduled to be taken is a medicine for which dietary restriction is set before taking it, and may be a fasting period in particular. The dietary restriction period setting unit 103 may set different dietary restriction periods according to the meal type information, and in particular, the dietary restriction period is set longer as the degree of meal indicated by the meal type information increases. More specifically, for example, in case that the meal type information is the high-fat meal, 8 hours is kept after the mealtime (i.e., the dietary restriction period is set to 8 hours), in case that the meal type information is the low-fat (normal) meal, 4 hours or more is kept after the mealtime (i.e., the dietary restriction period is set to 4 hours or more), and in case that the meal type information is the nutritional supplement, 2 hours is kept after the mealtime (i.e., the dietary restriction period is set to 2 hours).

An example of a drug for which the dietary restrictions are set is edaravone. The edaravone is 3-methyl-1-phenyl-2-pyrazolin-5-one represented by the following formula, and is one of medicines for treating ALS. When a pharmaceutical composition containing the edaravone as an active ingredient is orally or intragastrically administered, absorption rate is reduced by influence of meal. Therefore, it is preferable to inform a patient that the edaravone needs to be taken on an empty stomach in the morning (after overnight fasting), to stop eating at bedtime in the night before each administration, and to avoid eating and drinking other than water for 30 minutes or 1 hour after administration. If overnight fasting is not possible, it is preferable to fast for 8 hours before administration after the high-fat meal (1000 calories (synonymous with 1000 kcal as defined in the present specification), 50% fat) is ingested, and for 4 hours before administration after the normal meal (low-fat meal) (400 calories (synonymous with 400 kcal as defined in the present specification), 25% fat) is ingested. An alternative option for the patient may be, for example, the nutritional supplement (250 calorie (synonymous with 250 kcal as defined in the present specification) (eg, protein drink)) taken 2 hours prior to administration.

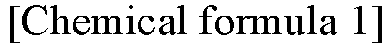

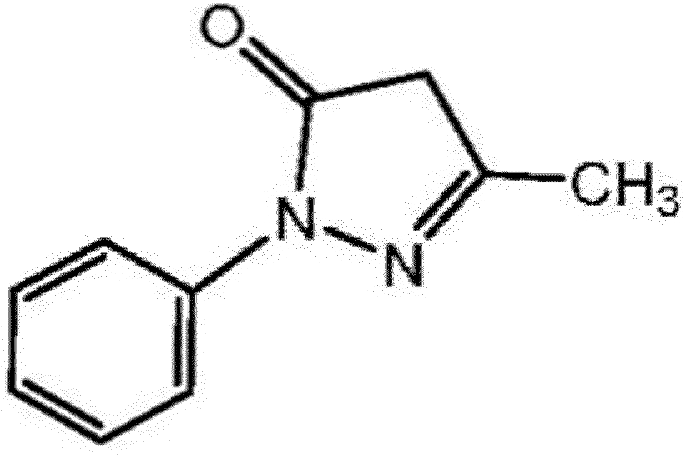

Then, based on the dietary restriction period information set by the dietary restriction period setting unit 103 and the mealtime information indicating the time when the predetermined meal is taken, the medication notification presentation unit 104 presents a medication notification to the first user terminal of the first user to encourage the first user to take medication at least one of the followings: a first time when the dietary restriction period has elapsed since the mealtime indicated by the mealtime information, a second time before the first time by a predetermined period (e.g., 5 minutes before the first time), and a third time after the first time by a predetermined period (e.g., 5 minutes after the first time). Setting for the medication notification at any of these times may be done by the selection operation by the user on the application of the first user terminal, or may be done in advance by a selection operation by a system provider or the second user. If information that medication has been taken (a medication taken information) is set by the input operation or the selection operation by the user on the application of the first user terminal, the medication notification may not be presented during a predetermined non-notification period (e.g., 24 hours).

In this way, by presenting the medication notification based on the dietary restriction period, it is possible to present the medication notification according to a medicine for which the dietary restriction period varies depending on the type of meal (meal content) especially.

The nutritional management target numerical value output unit 105 acquires physical information (e.g., height information, weight information, etc.) of the first user and self-reported information of the first user, for example, by the input operation or the selection operation by the user on the application of the first user terminal, calculates a recommended calorie intake (a recommended nutritional intake) suitable for the first user, and outputs nutritional management target numerical value information including the recommended calorie intake. The nutritional management target numerical value information is not limited to the recommended calorie intake, but may be a target value related to a recommended nutritional intake for other nutritional components, such as amount of lipid intake. Furthermore, the nutritional management target numerical value information may be, for example, an arbitrary numerical value input based on the past nutritional intake or the recommended nutritional intake, or a numerical value obtained by increasing or decreasing a corresponding predetermined value from the recommended nutritional intake in accordance with a predetermined amount of weight change (particularly, in accordance with a result of comparison with one or more reference change amounts).

The self-reported information may be self-reported information on the ALS Functional Rating Scale (ALSFRS-R). Here, the "ALSFRS-R" (ALS Functional Rating Scale-Revised) is used to assess daily living activities of an ALS patient, and is composed of total 12 items of evaluation items for limb motor dysfunction, bulbar dysfunction, and respiratory dysfunction. Each evaluation item is scored on a 5-point scale from 0 to 4 to evaluate overall severity and progression of disease in the ALS patient. The 12 items to be evaluated include 1) language, 2) saliva secretion, 3) swallowing, 4) writing, 5) eating behavior, 6) dressing/personal behavior, 7) behavior in bed, 8) walking, 9) climbing stairs, 10) dyspnea, 11) orthopnea, and 12) respiratory failure. In addition to the ALSFRS-R, other methods of assessing ALS include 40-item Amyotrophic Lateral Sclerosis (ALS) Assessment Questionnaire (ALSAQ-40), Japanese ALS Severity Classification, Modified Norris Scale, Rasch Overall ALS Disability Scale (ROADS), Communicative Participation Item Bank (CPIB), Neurological Fatigue Index-Motor Neuron(NFI-MND), ALS Depression Invention Inventory-12 (ADI-12), The ALS Specific Quality of Life-Revised (ALSSQOL-R), and ALS Cognitive Behavioral Screen (ALS-CBS).

The nutritional intake information generating unit 106 generates nutritional intake information indicating nutritional intake from the meal content information. More specifically, for example, nutritional intake information such as calorie intake information and fat intake information is generated by adding nutritional amounts such as calorie intake and fat intake corresponding to the meal content. Then, the comparison result generating unit 107 compares the nutritional intake information with the nutritional management target numerical value information (for example, recommended calorie intake information and recommended fat intake information) to generate comparison result information indicating the comparison result. The comparison result may be, for example, a result indicating difference between the recommended nutritional intake and the nutritional intake (i.e., a result indicating an excess or deficiency). The generated comparison result may be transmitted to the first user terminal 20 or the second user terminal 30 by the information managing unit 101.

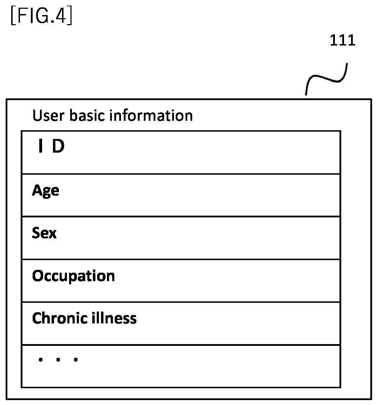

The user information storage unit **111** stores, for example, basic information of the user acquired by the information management unit 101. Fig. 4 shows an example of a configuration of user basic information stored in the user information storage unit 111. The user basic information may be associated with a user ID. The user basic information may include information on attributes such as user ID, user's age, sex, occupation and birth place, information on lifestyle habits such as chronic illness, medical history, allergies, constitution (obesity, frailty, etc.), dietary lifestyle, alcohol drinking, smoking and exercise habits, and self-reported information (for example, self-reported information for ALSFRS-R) , and may include name, address, height and weight of the user as necessary.

The generated information storage unit 112 stores, for example, generated information (such as the meal-related information, the dietary restriction period information, the nutritional management target numerical value information, the calorie intake information, and the comparison result information) generated in the various storage units.

### <Information processing flow>

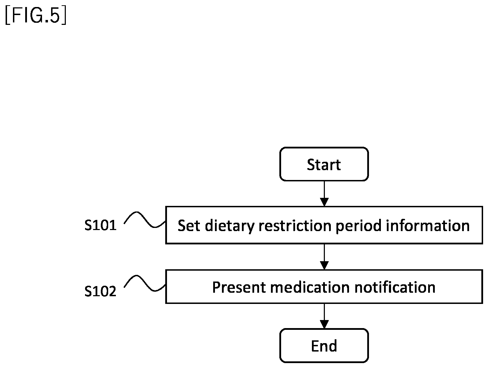

FIG. 5 is a diagram showing a flow of processing executed in the information processing apparatus 10 according to the present embodiment.

First, as a pre-processing step, the information management unit 101 of the information processing apparatus 10 accepts input of information about the user and stores the user basic information in the user information storage unit 111. If the user basic information has already been stored in the user information storage unit 111, it is also possible to refer to the user basic information required for the information processing according to the present embodiment by accepting input of the user ID or the like.

Next, the dietary restriction period setting unit 103 sets the dietary restriction period information indicating the dietary restriction period corresponding to the meal-related information indicating information related to meal content (S101).

Finally, based on the dietary restriction period information set by the dietary restriction period setting unit 103 and the mealtime information indicating the time when the predetermined meal is ingested, the medication notification presentation unit 104 presents a medication notification at least one among the first time when the dietary restriction period has elapsed from the mealtime indicated by the mealtime information, the second time before the first time by the predetermined period (e.g., 5 minutes before the first time), and the third time after from the first time by the predetermined period (S102), to the first user terminal of the first user encouraging the first user to take medication.

In this way, the information processing apparatus 10 of the present embodiment can present the medication notification based on the dietary restriction period, and can therefore present the medication notification according to the medicine for which the dietary restriction period varies depending on the type of meal (meal content) especially. Furthermore, the information processing apparatus 10 can also have with various management functions such as nutritional management.

Although the present embodiment has been described above, the above embodiment is intended to facilitate understanding of a present invention, and is not intended to limit the present invention. The present invention may be modified or improved without departing from a spirit of the present disclosure, and equivalents thereof are also included in the present invention.

For example, in the present embodiment, the information processing apparatus 10, the first user terminal 20, and the second user terminal 30 is illustrated as each one, but a plurality of first user terminals 20 and a plurality of second user terminals 30 can be connected to the information processing apparatus 10 via the network NW. The information processing apparatus 10 is described as a single computer, but this is not limited thereto, and may be configured as a system such that a functional unit and a storage unit are distributed among a plurality of computers. For example, each storage unit of the information processing apparatus 10 may be provided in a database server, and the information processing apparatus 10 may access the database server. Also, each functional unit may be provided in a distributed manner among a plurality of computers.

Furthermore, the present embodiment may include configurations other than the functional units and the components included in Fig. 3. Also, steps other than each step included in Fig. 5 may be added.

Although the preferred embodiment of the present disclosure has been described in detail above with reference to the attached drawings, technical scope of the present disclosure is not limited to such examples. A person having ordinary knowledge in a technical field of the present disclosure can conceive of various modified or amended examples within scope of the technical ideas described in the claims, and it is understood that these also naturally belong to the technical scope of the present disclosure.

In addition, effects described in the present specification are merely descriptive or exemplary and are not limiting. In other words, technology according to the present disclosure may achieve other effects that are apparent to a skilled person in the art from the description of the present specification in addition to or in place of the above effects.

- 1: information processing system
- 10: information processing apparatus
- 20: first user terminal
- 30: second user terminal
- NW: Network

## Claims

1. An information processing system comprises:
a dietary restriction period setting unit configured to set a dietary restriction period according to meal-related information regarding a predetermined meal of a first user, and
a medication notification presentation unit configured to present a medication notification based on mealtime information indicating mealtime when the predetermined meal is ingested and the dietary restriction period.

2. The information processing system according to claim 1, wherein the meal-related information includes at least one of a plurality of meal type information indicating degree of the predetermined meal.

3. The information processing system according to claim 2, wherein
the meal-related information includes meal content information indicating a content of the predetermined meal,
the meal content information is information of meal menu or ingredient set by an input operation or a selection operation by the first user or a related user related to the first user, or information of meal menu or ingredient estimated from an acquired image, and
the meal type information is set based on the information of meal menu or ingredient.

4. The information processing system according to claim 3, wherein the related user is at least one among a family member of the first user, a caregiver who cares for the first user, a medical professional involved with the first user, and a representative who acts on behalf of the first user.

5. The information processing system according to any one of claims 2 to 4, wherein the dietary restriction period setting unit sets the dietary restriction period according to the degree of the predetermined meal indicated by the meal type information.

6. The information processing system according to any one of claims 1 to 4, wherein the medication notification presentation unit presents the medication notification at a time of at least one among a first time when the dietary restriction period has elapsed from the mealtime indicated by the mealtime information, a second time before the first time by a predetermined period, and a third time after the first time by a predetermined period.

7. The information processing system according to any one of claims 1 to 4, further comprising a nutritional management target numerical value output unit configured to acquire physical information of the first user and ALSFRS-R evaluation information of the first user and output a nutritional management target numerical value including a recommended nutritional intake suited to the first user.

8. The information processing system according to claim 7, further comprising:
a nutritional intake information generation unit configured to generate nutritional intake information from a content of the predetermined meal, and
a comparison result generation unit configured to compare at least the nutritional intake information and the recommended nutritional intake to generate a comparison result.

9. The information processing system according to any one of claims 1 to 4, wherein the medication notification presentation unit does not present the medication notification during a predetermined non-notification period in case that medication taken information is set.

10. An information processing method executed on a computer, the method comprising:
a step of setting a dietary restriction period according to meal-related information related to a predetermined meal of a first user by a dietary restriction period setting unit, and
a step of presenting a medication notification based on mealtime information indicating mealtime when the predetermined meal is ingested and the dietary restriction period by a medication notification presentation unit.

11. A program for performing on a computer,
a dietary restriction period setting function for setting a dietary restriction period according to meal-related information related to a predetermined meal of a first user, and
a medication notification presentation function for presenting a medication notification based on mealtime information indicating mealtime when the predetermined meal is ingested and the dietary restriction period.
